(19) Europäisches Patentamt
European Patent Office
Office européen des brevets

(11) Veröffentlichungsnummer: **0 258 683**
**A2**

# (12) EUROPÄISCHE PATENTANMELDUNG

(21) Anmeldenummer: 87111493.0

(22) Anmeldetag: 08.08.87

(51) Int. Cl.4: **A61K 45/02**, A61K 9/70, A61K 47/00

(30) Priorität: 21.08.86 DE 3628468

(43) Veröffentlichungstag der Anmeldung: 09.03.88 Patentblatt 88/10

(84) Benannte Vertragsstaaten: AT BE CH DE ES FR GB GR IT LI LU NL SE

(71) Anmelder: Dr. Karl Thomae GmbH
Postfach 1755
D-7950 Biberach (Riss)(DE)

(72) Erfinder: Becker, Robert, Dr., Dipl.-Chem.
Hühnerfeldstrasse 26
D-7950 Biberach 1(DE)
Erfinder: Kruss, Bernd, Dr.
Birkenweg 6
D-7951 Hochdorf(DE)
Erfinder: Schilk, Leonhard
Wetterkreuzstrasse 23
D-7950 Biberach 1(DE)

## (54) Neue Applikationsformen für Alpha-Interferone.

(57) Beschrieben werden neue Applikationsformen für α-Interferone, insbesonders aber für Alpha-2-Interferon, bestehend aus einem nicht-lyophilisierten Trockenfilm aus säurestabilisierten α-Interferonen, z. B. Alpha-2-Interferon, auf einem inertem Träger, wobei die Träger vorzugsweise

a.) aus Augenstäbchen mit an den Enden gleichmäßig aufgebrachten Mengen an α-Interferonen für den ophthalmologischen Gebrauch zur Eintragung des Wirkstoffes in den Bindehautsack oder

b.) aus einer Trockenfilmampulle mit einem an den Innenrandungen haftenden Film der α-Interferone, für die Bereitung von Injektionslösungen, bestehen.

EP 0 258 683 A2

## Neue Applikationsformen für α-Interferone

Gegenstand der Erfindung sind neue Applikationsformen für α-Interferone, insbesonders aber für Alpha-2-Interferon, bestehend aus einem nicht-lyophilisierten Trockenfilm aus säurestabilisierten α-Interferonen, z. B. Alpha-2-Interferon, auf einem Träger aus Glas oder inertem Kunststoff, wobei die Träger vorzugsweise

a.) aus Augenstäbchen mit an den Enden gleichmäßig aufgebrachten Mengen an α-Interferonen für den ophthalmologischen Gebrauch zur Eintragung des Wirkstoffes in den Bindehautsack oder

b.) aus einer Trockenfilmampulle mit einem an den Innenran dungen haftenden Film der α-Interferone, für die Bereitung von Injektionslösungen, bestehen.

Bekanntlich sind die Interferone Proteine mit einem Molekulargewicht zwischen 15 und 30 kD, deren Aufbewahrung wegen ihrer Instabilität große Schwierigkeiten bereitet. Es sind in der Literatur bereits zahlreiche Vorschläge gemacht worden, interferonhaltige Präparate zu stabilisieren, beispielsweise wurde humanes Serumalbumin, Glucose, Mannitol und viele andere Verbindungen als Stabilisatoren verwendet. α- und γ-Interferone wurden auch für die Behandlung von Virenerkrankungen am Auge vorgesehen; es ist jedoch besonders schwierig, Salben bzw. Tropfen herzustellen, die über eine genügend lange Zeit hinweg keinen Wirkungsverlust erleiden.

Im japanischen Kokai Tokkyo Koho J.P. 55/102519 (80 102.519) wird eine Methode zur Stabilisierung von Interferon durch Gefriertrocknung in Gegenwart von Tris(hydroxymethylamino)-methan, eines nichtionogenen Polyethylensurfactants und eines Antibiotikums beschrieben. In der US-Patentschrift Nr. 4 252 791 wurde berichtet, daß Lanthanide und Calziumsalze die mechanische und thermische Stabilität der Interferone steigern. Durch die Europäische Patentanmeldung 82.481 ist es bekannt, daß Aminosäuren (z.B. Glycin und Alanin) gepuffertes (pH 7,0-7,4), lyophilisiertes Interferon stabilisieren. Nach Sedmak, J.J., et. al (Adv. Exp. Med. Biol. 1978, 110) ist Human-Fibroblasten-Interferon bei niederen pH-Werten nur in Gegenwart von mehr als 5 µg/ml Protein stabil, mechanischer Streß wirkt inaktivierend. Zusammenfassend läßt sich sagen, daß Interferone im allgemeinen und Alpha-2-Interferon im besonderen, in wässriger Lösung ohne Stabilisatoren sehr bald an Aktivität verlieren, auch Interferon-Reinlyophilisate, hergestellt durch Gefriertrocknung saurer, wäßriger Lösungen, sind nur dann relativ gut lagerstabil, wenn ihnen Stabilisatoren, z. B. Human-Serum-Albumine, und gegebenenfalls Puffersubstanzen, z. B. Ammoniumacetatpuffer, beigefügt sind.

Die für die ophthalmologische Anwendung in Frage kommenden Augenstäbchen sind Gegenstand der deutschen Patentschrift Nr. 2 441 191. Diese Augenstäbchen gestatten es, in der Ophthalmologie gebräuchliche Wirkstoffe in wohldosierter Weise zwischen dem unteren Augenlid und dem Augapfel einzutragen; genannt werden in dieser Patentschrift als Beispiele Fluorescein und Metoprolol für die Applikation am Auge. Mittels solcher Augenstäbchen kann eine genaue Dosierung vorgenommen werden, wobei es im Gegensatz zu Augentropfen zu keinen Irritationen am Auge kommt. Die Anwendung erfolgt in der Weise, daß das Ende des Stabchens unter das untere Augenlid gebracht und das Stäbchen zwei-bis dreimal gedreht wird; hierbei soll sich der Wirkstoff quantitativ ablösen.

Es wurde nun gefunden, daß nicht-lyophilisierte Trockenfilme aus protonierten α-Interferonen, insbesondere aus Alpha-2-Interferon, auf inerten Oberflächen, z. B. aus Glas oder Kunststoffen, haftend, eine ausgezeichnete Lagerstabilität aufweisen. Die nicht-lyophilisierten Trockenfilme werden dadurch hergestellt, daß das α-Interferon in einem sauren, stark polaren Lösungsmittel bei einem pH-Wert zwischen 1,5 und 5, vorzugsweise zwischen 3 und 4, quantitativ gelöst, die Lösung auf einen inerten Träger aus Glas oder inerten Kunststoffen (z.B. Polystyrol, Polyethylen, Polypropylen, Polycarbonat) aufgebracht und das Lösungsmittel bei Temperaturen zwischen 0°C und 80°C, vorzugsweise bei Zimmertemperatur, entfernt wird. Als Lösungsmittel eignen sich Wasser, aliphatische Alkohole mit 1 bis 4 Kohlenstoffatomen, aliphatische Ketone mit 3 bis 5 Kohlenstoffatomen oder Gemische dieser Lösungsmittel. Als Säuren zur Einstellung des pH-Wertes eignen sich anorganische Säuren wie Schwefelsäure, Phosphorsäure, insbesondere aber Salzsäure, als organische Säuren können z. B. Fumarsäure, Weinsäure, Bernsteinsäure oder Zitronensäure Verwendung finden.

Es ist besonders vorteilhaft, wenn das α-Interferon in einem azeotrop abdestillierbaren Gemisch aus Wasser und einem Alkohol, wie Methanol oder Ethanol, dem zuvor eine Säure, vorzugsweise Salzsäure, zur Einstellung des gewünschten pH-Wertes beigefügt wurde, gelöst wird, da nach dem Auftragen dieser Lösung auf den entsprechenden Träger auch die letzten Reste des vorhandenen Wassers leicht entfernbar sind. Da die Löslichkeit der α-Interferone in sauren, wäßrigen Lösungen am größten ist, empfiehlt es sich,

einer so hergestellten wäßrigen Lösung soviel Alkohol zuzugeben, daß das vorhandene Wasser vollständig zusammen mit dem Alkohol als ein azeotropes Gemisch bei Zimmertemperatur unter vermindertem Druck entfernt werden kann. Die Lagerstabilität des sich auf dem Träger ausbildenden Films ist größer, wenn der Feuchtegehalt dieses Films gering ist.

Vor der Beschichtung empfiehlt es sich, das Trägermaterial zu sterilisieren, desweiteren die Wirkstofflösung steril zu filtrieren. Man kann aber auch, auch als zusätzliche Maßnahme, das beschichtete Produkt anschließend durch Bestrahlung (z.B. mit ß-oder γ-Strahlen) sterilisieren.

Es ist überraschend, daß ein so erhaltener Trockenfilm aus einem protonierten α-Interferon auch bei mehrmonatiger Lagerung bei Zimmertemperatur noch fast seine volle Aktivität besitzt (selbst nach einer 5-tägigen Lagerung bei 60°C zeigt der Wirkstoff noch beinahe 90% seiner ursprünglichen Aktivität), während ein Film des gleichen Materials nach der Gefriertrocknung bereits ca. 40% seiner Ausgangsaktivität verloren hat. Selbst die Anwesenheit von Restspuren an Feuchtigkeit beeinträchtigt nicht die Stabilität des Wirkstoffes, was durch seine Protonierung bedingt wird.

Es ist auch überraschend, daß der erfindungsgemäß erhaltene Wirkstoff-Film sich mechanisch leicht ablöst, was für die Medikation am Auge sehr wichtig ist, und er sich in Wasser, noch besser in einer z. B. 0,1 N Salzsäurelösung, vor allem aber auch in der Tränenflüssigkeit sehr rasch auflöst, wodurch sich der Film sowohl für das Beschichten der Augenstäbchen als auch für das Befüllen von Ampullen gut eignet.

Im Gegensatz zu einer Gefriertrocknung arbeitet man bei der Herstellung der erfindungsgemäßen Applikationsformen in der Regel bei Zimmertemperatur, man braucht keine besonderen Kühlgeräte, man braucht aber auch keine weiteren Zusatz stoffe, wie z. B. Trägerstoffe und Stabilisierungsmittel, von denen man annahm, daß sie für die Herstellung lagerstabiler Interferon-Arzneimittelformen unerläßlich wären.

Die für die Ophthalmologie geeigneten Applikationsformen für α-Interferone, insbesondere für Alpha-2-Interferon, bestehen aus sogenannten Augenstäbchen (vgl. DE-B-2 441 191), bei welchen die dafür vorgesehenen Zonen, an einem oder an beiden Enden dieser Stäbchen, mit einer, dieses Interferon enthaltenden sauren, wäßrigen bzw. wäßrig-alkoholischen Lösung oder einer sauren, vorzugsweise Wasser enthaltenden Dialkylketon-, z. B. Aceton-Lösung rundum beaufschlagt wird unter sofortiger Entfernung des Lösungsmittels bis zur völligen Trockenheit des sich dabei bildenden Films.

Die zur Beaufschlagung der Augenstäbchen verwendeten Lösungen enthalten α-Interferon in einer Konzentration im allgemeinen zwischen 0,025 und 2 mg/ml, vorzugsweise zwischen 0,5 und 1,5 mg/ml. Die pro Augenstäbchen aufgebrachte Menge an α-Interferon beträgt zwischen 0,5 bis 50 µg, vorzugsweise 5 bis 20 µg (µg = Mikrogramm).

Das α-Interferon, z. B. rekombinantes Alpha-2-Interferon, das pulverförmig als Reinsubstanz vorliegt, wird in einer sauren Pufferlösung (z. B. in einer HCl-Glycin-Pufferlösung), vorzugsweise jedoch in Wasser oder in einer schwach salzsauren Lösung z. B. in einer 0,001 bis 0,1 N Salzsäure, aufgenommen bis zur Erreichung einer klaren Lösung. Die Lösung wird dann, beispielsweise durch Zugabe von Ethanol, auf die gewünschte Konzentration, z. B. Interferon-Konzentration von 0,5 mg/ml und Ethanol-Konzentration von 20 bis 90%, bevorzugt 70% (v/v), eingestellt. Hierbei wird ein systemspezifischer pH-Wert im allgemeinen von 1,5 bis 4,5 gemessen. Man kann zur Verbesserung der Haftung auf den Augenstäbchen der so erhaltenen Lösung noch eine Grundlage in einer Menge von 0,05 bis 2 Gew.-% zugeben, wobei die die Haftung auf den Augenstäbchen noch verbessernde Grundlage aus Stoffen wie Polyvinylalkohol, Polyvinylpyrrolidon oder Cellulosen, wie Methylcellulose, Hydroxypropylmethylcellulose, Carboxymethylcellulose, Hydroxyethylcellulose, hochmolekulare feste Polyethylenglykole oder Gemischen dieser Stoffe bestehen kann. Es können aber auch andere Grundlagen verwendet werden, sofern die Interferone darin stabil bleiben und diese Grundlagen gut flüssigkeitslöslich und augenverträglich sind. Bei einigen Trägermaterialien kann auf die Beimischung einer Grundlage ganz verzichtet werden; es kommt auf die Oberflächenbeschaffenheit der Trägermaterialien an, ob solche Beimischungen wünschenswert sind oder nicht. Die Grundlage dient, wie vorstehend bereits ausgeführt, als Haftmittel, sie vermag aber auch gleichzeitig die mechanische Filmstabilität günstig zu beeinflussen, zusätzlich läßt sich mit ihrer Hilfe auch die Viskosität der Beladelösung mitregulieren. Zur Verkürzung der Trocknungszeiten ist es von Vorteil, von möglichst hohen Konzentrationen des flüchtigen Lösungsmittels auszugehen.

Die Beaufschlagung bestimmter Zonen der Augenstäbchen kann nach den in der deutschen Patentschrift Nr. 2 441 191 beschriebenen Methode erfolgen, z. B. durch gesteuertes Eintauchen der Enden der Augenstäbchen in eine ein Interferon enthaltende erfindungsgemäße Lösung. Das Eintauchverfahren bedingt aber einen nicht unerheblichen Verlust an teurem Wirkstoff. Es empfiehlt sich deshalb, die Wirkstofflösung nach der in der deutschen Patentanmeldung P 35 13 288.4 beschriebenen Methode auf die

vorgesehenen Zonen der rotierenden Augenstäbchen berührungslos aufzupunkten, wobei gleichzeitig oder anschließend das Lösungsmittel verdampft wird. Das Verdampfen erfolgt vorzugsweise dadurch, daß ein 40 bis 80°C warmer Strom eines sterilen Gases, z. B. Luft, auf die rotierenden Zonen der Stäbchen gerichtet wird.

Für die Herstellung von α-Interferon enthaltenden Trockenampullen wird die saure, Interferon enthaltende Lösung nach ihrer Sterilfiltrierung in geeignete, sterile Behälter aus Kunststoffmaterial oder Glas eingefüllt. Das Lösungsmittel wird bei Zimmertemperatur oder bei Temperaturen bis zu 50°C unter vermindertem Druck langsam entfernt; so werden beispielsweise nach dem Befüllen der Behälter diese durch langsames Evakuieren abgekühlt und anschließend langsam unter Vakuum auf Zimmertemperatur gebracht, bis das gesamte Lösungsmittel entfernt ist, bevorzugt wendet man aber eine Tiefentemperatur-Destillation an, wobei das Einfrieren der Lösung zu verhindern ist. Die so beschichteten Behälter, z. B. Ampullen, werden anschließend dichtgemacht, z. B. durch Zuschmelzen des Glashalses. Vor der Applikation wird der Inhalt der Trockenampulle in einer sterilen isotonischen Lösung gelöst.

Bei den bisher angewandten Methoden zur Stabilisierung von Alpha-2-Interferon-Zubereitungen bediente man sich sogenannter Stabilisatoren; ein bevorzugter Stabilisator war z. B. humanes Serumalbumin (HSA). Selbst wenn man die Gefriertrocknung durch die erfindungsgemäße Trocknungsmethode ersetzt, erhält man in Gegenwart solcher Stabilisatoren eine schlechtere Lagerstabilität als in Abwesenheit dieser Hilfsstoffe. Dies geht aus den folgenden Beobachtungen hervor:

Eine saure Lösung von Alpha-2-Interferon, hergestellt aus 5 mg Alpha-2-Interferon, welche in 3 ml 0,003 n Salzsäure zusammen mit 10 mg HSA und 25 mg Methocel gelöst wurden, wurde mit 7 ml Ethanol versetzt (pH-Wert der Lösung 3,6), sterilfiltriert und jeweils auf ein Ende der rotierenden Augenstäbchen aufgepunktet unter gleichzeitiger Entfernung des Lösungsmittels durch Überleiten eines sterilen Luftstromes bei 60°C. Pro Augenstäbchen wurden 10 µl der Beladelösung aufgepunktet, jedes Stäbchen trug damit 5 µg Alpha-2-Interferon = Stäbchen A.

Eine erfindungsgemäße saure Lösung von Alpha-2-Interferon, hergestellt aus 5 mg Alpha-2-Interferon, gelöst in 3 ml 0,0015 n Salzsäure und versetzt mit 7 ml Ethanol (pH-Wert der Beladelösung 4,2) wurde in analoger Weise auf die Enden der Augenstäbchen gebracht. Auch hier trug jedes Stäbchen eine Menge von 5 µg Alpha-2-Interferon = Stäbchen B.

Die Stäbchen A und B wurden vergleichend in Bezug auf ihre Lagerstabilität einmal durch 48-stündige Lagerung bei 60°C, das anderemal durch eine 1-monatige Lagerung bei 41°C untersucht. Hernach wurde die Aktivität des gelagerten Wirkstoffes bestimmt (ELISA-Testmethode) und in % bezogen auf die Anfangsaktivität (= 100%) ausgedrückt. Die folgende Tabelle enthält die dabei gefundenen Werte:

| | Aktivität des Wirkstoffes nach einer Lagerungszeit und -Temperatur von | |
|---|---|---|
| | 48 Stunden / 60°C | 1 Monat / 41°C |
| Stäbchen A | 81,4 % | 69,8 % |
| Stäbchen B | 94,0 % | 84,0 % |

Die erfindungsgemäßen Hilfsstoff-freien Applikationsformen sind also deutlich lagerungsstabiler als entsprechende durch Zusatzstoffe stabilisierte Formen. Wie bereits beschrieben, sind die Wirkstoff-Aktivitäten gefriergetrockneter Formen noch wesentlich niedriger (vgl. Angaben hierzu auf der Seite 4).

Die nachfolgenden Beispiele dienen zur näheren Erläuterung des Gegenstandes der Erfindung (µg = Mikrogramm, µl = Mikroliter):

Beispiel 1

Augenstäbchen

50 mg pulverförmiges, rekombinantes Aloha-2-Interferon wurden in 10 ml 0,01 n Salzsäure aufgenommen, die Lösung mit 90 ml reinem Ethanol versetzt. Zu dieser Lösung wurden 0,5 g Hydroxypropylmethylcellulose zugegeben. Die so erhaltene Lösung (pH-Wert 3,5) wurde steril filtriert, jeweils 10 µl wurden auf ein Ende von sterilen, rotierenden Augenstäbchen aufgetropft und gleichzeitig in einem Strom steriler Luft von 60°C getrocknet.

Die pro Augenstäbchen aufgetropfte Lösungsmenge enthält 5 µg Alpha-2-Interferon.

Die Stäbchen wurden 5 Tage bei 60°C gelagert und hernach der Wirkstoffgehalt mit 85%, bezogen auf den Anfangswert, bestimmt.

In analoger Weise wurden in den nachfolgenden Beispielen 2 bis 6 Augenstäbchen mit einer sauren Lösung von Alpha-2-Interferon der folgenden Zusammensetzung beaufschlagt:

| | | Beispiele | | | | |
|---|---|---|---|---|---|---|
| Stoff | Menge | 2 | 3 | 4 | 5 | 6 |
| Alpha-2-Interferon | mg/ml | 0,5 | 0,5 | 1,5 | 1,0 | 1,5 |
| Methanol | % v/v | - | - | - | 50 | - |
| Ethanol | % v/v | 70 | 90 | 70 | - | 70 |
| Salzsäure | ad pH | 3,5 | 3,0 | 4,2 | 4,0 | 3,5 |
| Methylcellulose | Gew.-% | - | - | - | 0,2 | - |
| Polyvinylalkohol | Gew.-% | - | - | - | - | 2 |

Das Beladevolumen pro Augenstäbchen, einseitig beaufschlagt, betrug bei den Beispielen 2 bis 6 jeweils 10 µl. Die Augenstäbchen bestanden aus Glas und Polystyrol.

Beispiel 7

Alpha-2-Interferon enthaltende Trockenampullen

20 mg pulverförmiges, rekombinantes Alpha-2-Interferon wurde in 4 ml 0,1 n Salzsäure aufgenommen, die Lösung wurde mit 36 ml reinem Ethanol versetzt, pH-Wert der Lösung 2,7. Die so erhaltene Lösung wurde steril filtriert; von dieser Lösung wurden jeweils 40 µl in eine sterilisierte Glasampulle abgefüllt. Wirkstoffinhalt pro Ampulle: 20 µg. Anschließend ließ man die Ampullen durch Anlegen eines Vakuums langsam abkühlen, die restlichen Lösungsmittelmengen wurden nach Erwärmen der Ampullen auf Zimmertemperatur oder Temperaturen bis 40°C im Vakuum entfernt. Die einen Trockenfilm enthaltenden Ampullen wurden in an sich üblicher Weise luftdicht verschlossen.

In analoger Weise wurden in den nachfolgenden Beispielen 8 bis 11 Ampullen mit 30 µl einer sauren Lösung von Alpha-2-Interferon der folgenden Zusammensetzung beschickt:

| Stoff | Volumen- bzw. Mengenverhältnis | | Beispiele 8 | 9 | 10 | 11 |
|---|---|---|---|---|---|---|
| Alpha-2-Interferon-Konzentr. | mg/ml | | 0,5 | 1,0 | 1,0 | 0,5 |
| Ethanol | % v/v | | 70 | - | 50 | - |
| Methanol | % v/v | | - | 85 | - | - |
| Salzsäure | | ad pH | 3,0 | 2,5 | - | 3,5 |
| Schwefelsäure | | ad pH | - | - | 3,8 | - |
| µg IFN/30 µl = Trockensubstanz pro Ampulle | | | 15 | 30 | 30 | 15 |

Im Beispiel 11 diente nur Wasser als Lösungsmittel.

Beispiel 12

Augenstäbchen

Rezeptur folgender Zusammensetzung pro 1 Augenstäbchen:

Alpha-2-Interferon 5 µg
Polyvinylpyrrolidon 25 µg
Zitronensäure × $H_2O$ 2,5 µg
$H_2O$/Ethanol (70%, v/v) ad 10 µl

Die Herstellung der Lösung und Beaufschlagung der Augenstäbchen erfolgte wie in Beispiel 1 beschrieben.

Beispiel 13

Augenstäbchen

Rezeptur folgender Zusammensetzung pro 1 Augenstäbchen:

Alpha-2-Interferon 8 µg
Hydroxypropylmethylcellulose 10 µg
0,001 n Salzsäure/Ethanol
(50%, v/v) ad 10 µl

Die Herstellung der Lösung und Beaufschlagung der Augenstäbchen erfolgte wie in Beispiel 1 beschrieben.

Beispiel 14

Augenstäbchen

Rezetur folgender Zusammensetzung pro 1 Augenstäbceh:

Alpha-2-Interferon 10 µg
Polyvinylalkohol 50 µg
0,01 n Salzsäure/Ethanol
(70%, v/v) ad 10 µl

Die Herstellung der Lösung und Beaufschlagung der Augenstäbchen erfolgte wie in Beispiel 1 beschrieben.

**Ansprüche**

1.) Applikationsformen für α-Interferone, bestehend aus einem nicht-lyophilisierten Trockenfilm aus säurestabilisierten α-Interferonen auf einem Träger aus Glas oder inertem Kunststoff.

2.) Applikationsformen gemäß Anspruch 1, dadurch gekennzeichnet, daß ein nicht-lyophilisierter Trockenfilm aus säurestabilisierten α-Interferonen auf einem Träger in Form von

a.) einem Augenstäbchen an den Enden dieser Augenstäbchen gleichmäßig oder

b.) einer Trockenampulle als ein an deren Innenwandungen haftender Film aufgebracht ist.

3.) Applikationsformen gemäß Anspruch 1 und 2, dadurch gekennzeichnet, daß als Wirkstoff Alpha-2-Interferon in Form eines Trockenfilmes vorliegt.

4.) Applikationsformen gemäß den Ansprüchen 1 bis 3, dadurch gekennzeichnet, daß der Trockenfilm noch eine die Haftung fördernde Grundlage enthält.

5.) Applikationsformen gemäß den Ansprüchen 1 bis 4, dadurch gekennzeichnet, daß der pro Augenstäbchen aufgebrachte Film 0,5 bis 50 Mikrogramm α-Interferon enthält.

6.) Applikationsformen gemäß Anspruch 4, dadurch gekennzeichnet, daß die die Haftung fördernde Grundlage aus Polyvinylalkohol, Polyvinylpyrrolidon, hochmolekulare feste Polyethylenglykole, oder Cellulosen, wie Methylcellulose, Hydroxypropylmethylcellulose, Hydroxyethylcellulose, Carboxymethylcellulose oder aus Gemischen dieser Stoffe besteht.

7.) Verfahren zur Herstellung neuer Applikationsformen für α-Interferone, dadurch gekennzeichnet, daß die α-Interferone in einem sauren, stark polaren Lösungsmittel bei einem pH-Wert zwischen 1 und 5 quantitativ gelöst, die Lösung, gegebenenfalls steril filtriert, auf einen inerten Träger aufgebracht und das Lösungsmittel bei Temperaturen zwischen 0 und 80°C entfernt wird.

8.) Verfahren gemäß Anspruch 7, dadurch gekennzeichnet, daß als stark polare Lösungsmittel Wasser, aliphatische Alkohole mit 1 bis 4 Kohlenstoffatomen, aliphatische Ketone mit 3 bis 5 Kohlenstoffatomen oder Gemische dieser Lösungsmittel verwendet werden und die Volumenverhältnisse bei den wasserhaltigen Gemischen so gewählt werden, daß sich diese als azeotrope Gemische abdestillieren lassen.

9.) Verfahren gemäß Anspruch 7 und 8, dadurch gekennzeichnet, daß als anorganische Säuren Schwefelsäure, Phosphorsäure, insbesondere Salzsäure, als organische Säuren Fumarsäure, Weinsäure, Bernsteinsäure oder Zitronensäure zur Herstellung eines sauren, polaren Lösungsmittels verwendet werden und damit ein pH-Wert von 2 bis 4 eingestellt wird.

10.) Verfahren gemäß Anspruch 7 bis 9, dadurch gekennzeichnet, daß eine ein α-Interferon enthaltende saure, wäßrige bzw. wäßrig-alkoholische oder eine saure, wäßrige Dialkylketon-Lösung

a.) an einem oder an beiden Enden von Augenstäbchen rundum beaufschlagt wird unter sofortiger Entfernung des Lösungsmittels bis zur völligen Trockenheit des sich bildenden Films, oder

b.) in Trockenampullen aus Kunststoffmaterial oder Glas eingefüllt und das Lösungsmittel bei Temperaturen bis 80°C unter vermindertem Druck und unter Vermeidung eines Gefriervorganges entfernt wird, wobei anschließend die Ampullen verschlossen werden,

gegebenenfalls nach vorheriger Sterilfiltrierung der eingesetzten Lösungen und Sterilisierung der Träger.

Claims for the following Contracting States : AT, GR, ES

1.) Verfahren zur Herstellung neuer Applikationsformen für α-Interferone, dadurch gekennzeichnet, daß die α-Interferone in einem sauren, stark polaren Lösungsmittel bei einem pH-Wert zwischen 1 und 5 quantitativ gelöst, die Lösung, gegebenenfalls steril filtriert, auf einen inerten Träger aufgebracht und das Lösungsmittel bei Temperaturen zwischen 0 und 80°C entfernt wird.

2.) Verfahren gemäß Anspruch 1, dadurch gekennzeichnet, daß als stark polare Lösungsmittel Wasser, aliphatische Alkohole mit 1 bis 4 Kohlenstoffatomen, aliphatische Ketone mit 3 bis 5 Kohlenstoffatomen oder Gemische dieser Lösungsmittel verwendet werden und die Volumenverhältnisse bei den wasserhaltigen Gemischen so gewählt werden, daß sich diese als azeotrope Gemische abdestillieren lassen.

3.) Verfahren gemäß Anspruch 1 und 2, dadurch gekennzeichnet, daß als anorganische Säuren Schwefelsäure, Phosphorsäure, insbesondere Salzsäure, als organische Säuren Fumarsäure, Weinsäure, Bernsteinsäure oder Zitronensäure zur Herstellung eines sauren, polaren Lösungsmittels verwendet werden und damit ein pH-Wert von 2 bis 4 eingestellt wird.

4.) Verfahren gemäß Anspruch 1 bis 3, dadurch gekennzeichnet, daß eine ein $\alpha$-Interferon enthaltende saure, wäßrige bzw. wäßrig-alkoholische oder eine saure, wäßrige Dialkylketon-Lösung

a.) an einem oder an beiden Enden von Augenstäbchen rundum beaufschlagt wird unter sofortiger Entfernung des Lösungsmittels bis zur völligen Trockenheit des sich bildenden Films, oder

b.) in Trockenampullen aus Kunststoffmaterial oder Glas eingefüllt und das Lösungsmittel bei Temperaturen bis 80°C unter vermindertem Druck und unter Vermeidung eines Gefriervorganges entfernt wird, wobei anschließend die Ampullen verschlossen werden,

gegebenenfalls nach vorheriger Sterilfiltrierung der eingesetzten Lösungen und Sterilisierung der Träger.